# EUROPEAN PATENT SPECIFICATION

(11) **EP 4 469 789 B1**
(45) Date of publication and mention of the grant of the patent: **02.12.2026**
(21) Application number: 23705648.6
(22) Date of filing: 23.01.2023
(51) Int. Cl.: G01N 21/88, G01N 33/12, G01N 21/84, G01N 33/02

(54) **DEVICE FOR THE INSPECTION AND FOR THE SELECTION OF FOOD PRODUCTS CONTAMINATED BY AFLATOXINS**
VORRICHTUNG ZUR INSPEKTION UND AUSWAHL VON MIT AFLATOXINEN KONTAMINIERTEN NAHRUNGSMITTELN
DISPOSITIF D'INSPECTION ET DE SÉLECTION DE PRODUITS ALIMENTAIRES CONTAMINÉS PAR DES AFLATOXINES

(30) Priority: 24.01.2022 IT 202200001121
(43) Date of publication of application: 04.12.2024
(73) Proprietor: Di Bartolo Srl, 95011 Calatabiano (CT) (IT)
(72) Inventor: DI BARTOLO, Rosario, 95011 Calatabiano (CT) (IT)
(74) Representative: Fisauli, Beatrice A. M.
(86) International application number: PCT/IB2023/050542
(87) International publication number: WO 2023/139550

(56) References cited:
- EP-A1- 2 270 475
- WO-A1-2019/201786
- CN-B- 104 646 315
- GB-A- 2 388 764
- US-A1- 2004 134 504
- US-A1- 2009 311 392
- US-A1- 2012 093 985

## Description

The present invention concerns an apparatus for the inspection and selection of food products potentially contaminated by aflatoxins, in particular solid bulk foods such as cereals, pulses and dried fruit, more specifically pistachios.

The present invention also concerns a system for the treatment of these food products contaminated by aflatoxins, equipped with the aforesaid inspection and selection apparatus.

As is known, aflatoxins are produced by the metabolism of some fungal strains of Aspergillus flavus and Aspergillus parasiticus, which develop in numerous plants such as cereals, oilseeds (for example peanuts), spices, grains, dried fruit and nuts, both during harvesting and during storage.

Among the aflatoxins known to date, only some are considered important both for diffusion and for toxicity, namely the aflatoxins B1, B2, G1, G2 and M1. Among these, the most toxic aflatoxin is the aflatoxin B1, which is commonly considered genotoxic and hepatocarcinogenic.

The effects on the human body caused by the consumption (or assimilation) of non-negligible doses of aflatoxins, in particular of the aflatoxin B1, are well known, and include immune system disorders, liver problems, and in some cases, even death.

For these reasons, European law, in particular EU regulation 165/2010, establishes the maximum levels of aflatoxins tolerable in food products such as cereals, dried fruit and milk.

Among the products subject to contamination by the aflatoxin B1 pistachios are those that have the highest level of risk. Specifically, although the incidence of contamination in these products is relatively low, a single fruit can carry a very large amount of aflatoxins, at times tens of orders of magnitude higher than the level established by law per kilogram of product.

Normally, sample checks are carried out on these products. However, due to the low incidence of contamination by aflatoxins, the conventional control system does not guarantee sufficient safety for consumers of these foods or of processed food products that contain pistachios.

Document US 2012/093985 A1 discloses a nondestructive system and method for detecting aflatoxin contamination in produce, particularly nuts such as almonds, using red-orange fluorescence. The system illuminates the produce on a sorting plane with a wide-band black light (UV), captures images with an RGB camera, and evaluates the red channel of the images to identify contaminated items. Contaminated produce is detected based on elevated red channel values (corresponding to fluorescence in the 620-720 nm range) and is automatically removed from the sorting line.

Document WO 2019/201786 A1 relates to an optical sorting machine for granular agricultural products (e.g., maize, rice, peanuts) that detects aflatoxin-contaminated kernels by inducing fluorescence with UV illumination and measuring the emitted light in two wavelength ranges ( blue 450-495 nm and green 495-570 nm). The document mentions that belt- type transport processes could be used. In an embodiment a bichromatic camera or alternatively a hyperspectral cameras can be used for the inspection.

Document US 2009/311392 A1 discloses a method and apparatus for the simultaneous decontamination and detoxification of solid foodstuffs such as nuts, grains, fruits, and vegetables. The document describes a system where food detected as contaminated is sorted out (diverted) for appropriate treatment, while non-contaminated food can bypass unnecessary processing. This is achieved through real-time monitoring (such as by IR inspection or by UV fluorescence inspection) and through sortation.

In this context, the object of the present invention is to propose an apparatus for the inspection and selection of food products contaminated by aflatoxins, which overcomes the aforesaid problems of the prior art.

In particular, an object of the present invention is to produce an apparatus capable of inspecting solid bulk food products, such as dried fruit or cereals, of identifying potentially contaminated products and of separating them from the uncontaminated products, hereinafter also indicated as "good" products.

A further object of the present invention is to provide an in-line apparatus capable of processing high volumes of product and, therefore, of allowing a company to carry out non-destructive inspection of all product batches to be placed on the market or to be used as ingredients to prepare recipes or semi-finished products.

Another object of the present invention is to produce an apparatus capable of identifying products contaminated by aflatoxins with high precision, in particular of distinguishing products effectively contaminated from so-called "false positive" products, i.e., those carrying on the surface substances that are fluorescent in a wavelength comparable with that of the aflatoxins, such as kojic acid, the metabolite of the fungus that produces aflatoxin, which, per se, is not toxic.

Yet another object of the present invention is to produce an in-line system for treating food products contaminated, or potentially contaminated, by aflatoxins and reintroducing them into the production cycle.

These objects are achieved by an apparatus for the inspection and selection of food products contaminated by aflatoxins, in particular cereals and dried fruit, in conformity with claim 1.

In particular, according to the invention, the apparatus comprises at least:
- a conveyor line;
- a first inspection station; and
- a first selection unit.

The conveyor line is configured to transfer the food products between an inlet of the apparatus and at least one outlet of the apparatus; said conveyor line has a supporting surface suitable to receive the solid bulk food products distributed in a uniform layer.

The first inspection station is configured to inspect a flow of food products in movement carried by the conveyor line. According to the invention, said first inspection station comprises:
- a first housing positioned above the supporting surface of the conveyor line;
- at least one ultraviolet light source, mounted in the first housing so as to direct a beam of ultraviolet light toward the food products on the supporting surface;
- an RGB video camera mounted in the first housing and oriented toward the area of the supporting surface illuminated by the ultraviolet source;
- a first control unit connected at least to the RGB video camera.

According to the invention, said control unit is configured to receive and analyse the images filmed by the video camera, in particular to identify in the layer of food products in movement on the conveyor line those with photoemissions (so-called induced fluorescence) that indicate the presence of aflatoxins, i.e., that are potentially contaminated.

The first selection unit, located downstream of the first inspection unit, is controlled by the first control unit and is configured to separate the good food products from those that are potentially contaminated and to eject the former toward a collection point.

Therefore, with the apparatus according to the present invention it is possible to accurately inspect large amounts of food products in real time and to identify the single contaminated products and separate them from the good products in an automated manner.

According to an aspect of the invention, the first inspection unit comprises a pair of ultraviolet light sources arranged in the housing facing each other, relative to the direction of movement of the conveyor line, and oriented so that the light beam of each is directed inclined relative to the supporting surface of the conveyor line with an angle between 35° and 55° (or 125° and 145° considering the complementary angle). In this way, it is possible expose the surface of the food products to the ultraviolet light source in a uniform and complete manner, eliminating or minimising the "grey area".

The RGB video camera is mounted in the housing preferably in a central position between the two ultraviolet light sources, and is preferably oriented substantially normal to the supporting surface of the conveyor line.

According to another aspect of the invention, the at least one ultraviolet light source is configured to emit light with a wavelength of 365 nm. This wavelength is particularly effective for stimulating the fluorescence of the aflatoxin and therefore facilitates its identification by an algorithm implemented in the control unit.

According to the invention, the apparatus also comprises a second inspection unit, placed downstream of the first selection unit along the conveyor line, suitable to inspect the flow of potentially contaminated food products identified by the first inspection unit.

According to the invention, the second inspection unit comprises:
- a second housing positioned above the supporting surface of the conveyor line;
- at least one ultraviolet light source, mounted in the second housing so as to direct a beam of ultraviolet light toward the product on the supporting surface;
- at least one hyperspectral video camera, mounted in the second housing and oriented toward the area of the supporting surface illuminated by the ultraviolet source;
- a second control unit connected at least to the hyperspectral video camera.

The second control unit is configured to receive the images filmed by the at least one hyperspectral video camera and to analyse said images, in particular to distinguish, based on the spectrum of the photoemissions of said products, those probably contaminated by aflatoxins from the good products.

According to the invention, the apparatus further comprises a second selection unit, downstream of the second inspection unit, controlled by the second control unit and configured to separate the uncontaminated food products from those probably contaminated and to eject the former toward a collection point.

According to an aspect of the invention, the second inspection unit can comprise two or more hyperspectral video cameras.

According to this variant, each video camera is set to detect a specific wavelength or a predefined range of wavelengths.

According to a preferred embodiment, a first video camera is set to detect photoemissions with wavelengths between 430 nm and 450 nm, a second video camera is set to detect photoemissions with wavelengths between 480 nm and 500 nm, and a third video camera is set to detect photoemissions with wavelengths between 520 nm and 540 nm.

This configuration allows effectively contaminated products, in which photoemissions are between 430 nm and 450 nm, to be distinguished from false positives due to the presence of kojic acid, with photoemissions between 480 nm and 500 nm or natural photoemissions of the pistachio between 520 nm and 540 nm.

The video cameras are configured to operate in parallel and each of them can be associated with second independent control units, or with a single control unit.

The various control units of the apparatus can be implemented and managed from a control station of the apparatus.

This control station is typically equipped with viewing interfaces (screens, displays, etc.) and input interfaces (keyboards, touch screen displays or similar). According to a preferred variant, the video camera of the first inspection unit and each of the hyperspectral video cameras of the second inspection unit are connected to a viewing interface that allows viewing of the images filmed or other operating parameters such as graphics or similar.

According to a preferred embodiment, the second inspection unit comprises at least one ultraviolet light source arranged in the housing above the belt and oriented so that the light beam is substantially orthogonal to the supporting surface. The hyperspectral video cameras are preferably arranged so that the observation point is perpendicular to the supporting surface.

Also in this case, the ultraviolet light source is configured to emit light with a wavelength of 365 nm.

The aforesaid objects are also achieved with a system for treating food products, in particular cereals and dried fruit, contaminated by aflatoxins.

According to the invention, the system comprises an inspection and selection apparatus according to one of the variants described above, which delivers at the outlet a flow of food products potentially or probably contaminated distributed in a uniform layer on the conveyor line.

In detail, this flow of potentially or probably contaminated products comes from the first selection unit, where it is the only one present in the apparatus, or from the second selection unit, which has further treated the separated flow of food products delivered from the first selection unit.

According to the invention, the system comprises at least one first treatment unit to eliminate or reduce the aflatoxins present in the food products. Said first treatment unit is typically positioned above the conveyor line, downstream of the inspection and selection apparatus, and comprises one or more ultraviolet light sources in the UV-C band, i.e., with a wavelength between 100 nm and 280 nm, oriented to direct at least a part of the light beam onto the layer of food products.

According to a preferred embodiment, the UV-C ultraviolet light sources comprise tube lamps arranged with their axis extending substantially parallel to the direction of movement of the food products on the conveyor line.

The number of lamps and their length depends on various factors, such as the extension in width of the supporting surface of the products and the average speed at which they are conveyed on the conveyor line.

To maximise the efficacy in destroying aflatoxins, the UV-C ultraviolet light sources are placed at a distance from the supporting surface preferably between 15 mm and 50 mm. For this purpose, the apparatus comprises a device, preferably placed downstream of the first inspection unit, that distributes the products in a uniform layer preventing areas of accumulation. This operation also allows better viewing of the products in the inspection unit.

According to another aspect of the invention, the system can comprise a second treatment unit, downstream of the first treatment unit, which comprises a plurality of ozonator devices, positioned along the conveyor line, suitable to generate a flow of ozone directed toward the layer of food products.

Said ozonator devices are preferably positioned in a containment chamber in the form of a tunnel placed above the conveyor line and in direct communication with the food products. Said chamber is also in communication with a suction device configured to draw in the excess gas and prevent it from dispersing outside the unit.

The action of these ozonator devices allows an increase in the efficacy in destroying aflatoxins, especially in foods such as pistachios, the partially open shell of which can obstruct (shield) the action of the ultraviolet radiation. On the contrary, the gases produced by the ozonator devices can surround the products completely, even reaching the interstices between the surface of the nut and the shell.

The positions of the first treatment unit and of the second treatment unit can also be inverted relative to the direction of flow of products; the second treatment unit can be directly downstream of the inspection apparatus.

According to another aspect of the invention, the second treatment unit can comprise an ionising radiation emitting device oriented toward the flow of food products to be treated. This device allows the destruction of aflatoxins, and other pathogenic species, also under the surface of the food product, guaranteeing an effective sanitising action also for products in which the outer shell is only slightly open.

More in detail, according to this variant, said second treatment unit comprises an X-ray tube that generates radiation in the X-ray spectrum. The power used by the X-ray tube is preferably greater than 500 W and more preferably greater than 700 W, while the voltage is at least 30,000 V, preferably at least 50,000, typically up to 90,000 V.

According to another aspect of the invention, the system can comprise at least one video camera configured to film the flow of food products entering the treatment unit, i.e., downstream of the selection devices. According to this variant, at least one of the control units for controlling the treatment unit is connected to said video camera and is configured to control one or more of said treatment units in real time as a function of the amount of food product present on the conveyor line.

In particular, thanks to this system for controlling the flow of contaminated products in real time, it is possible to selectively exclude the treatment units or sub-parts thereof (for example, only some of the UV-C lamps or some of the ozonator devices) or in any case to modify its action, for example by varying the power supplied.

This video camera is preferably arranged immediately upstream of the first treatment unit after the inspection block, or directly inside one of said units.

Further features and advantages of the present invention will be more apparent from the description of a preferred, but not exclusive, example of embodiment of a system for the treatment of food products, as illustrated in the accompanying figures, wherein:
- Fig. 1 is a side view of the system for the treatment of the food products according to the present invention;
- Fig. 2 is a perspective view of the first inspection unit partially disassembled;
- Fig. 3 is a perspective view of the second inspection unit partially disassembled;
- Fig. 4 is a perspective view of the selection unit;
- Fig. 5 is a perspective view of the first treatment unit partially disassembled;

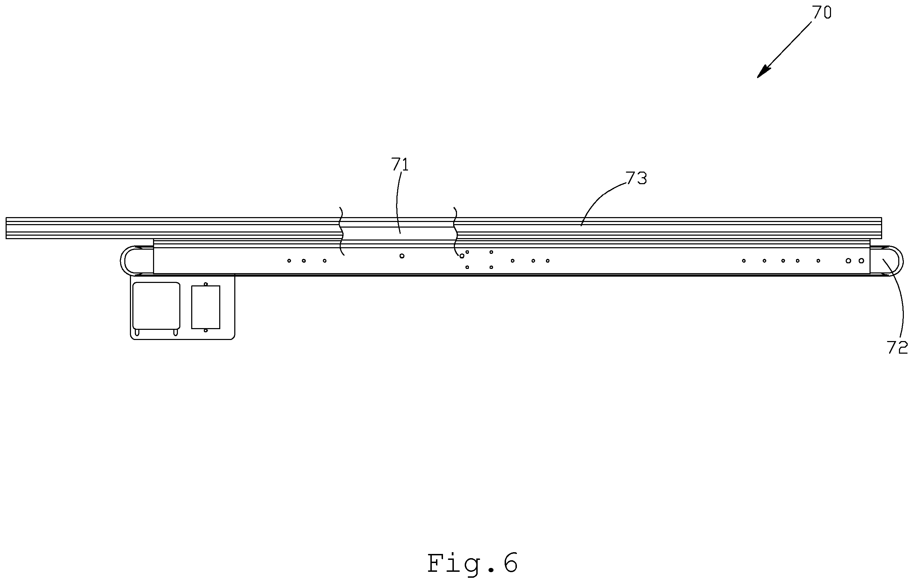

- Fig. 6 is a side view of the second treatment unit partially disassembled;

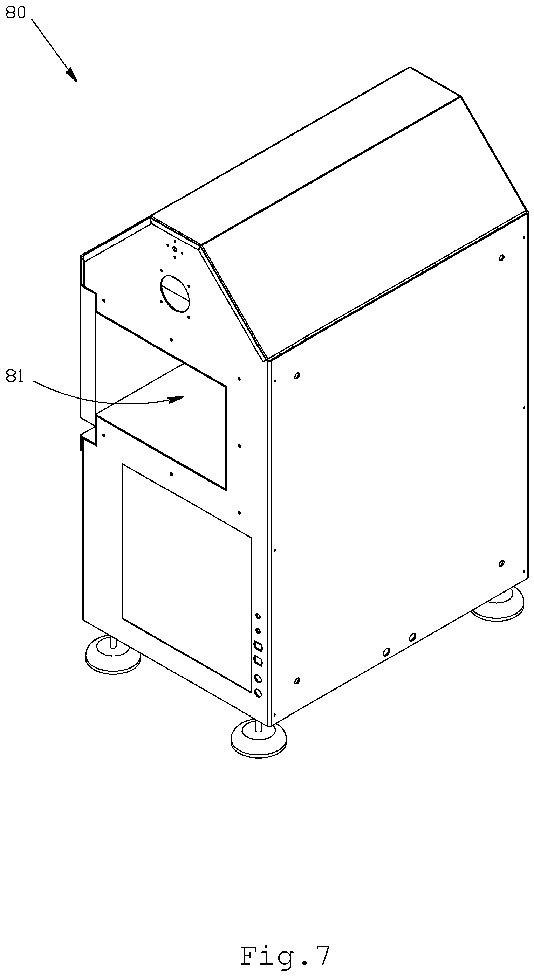

- Fig. 7 is a perspective view of the third treatment unit partially disassembled.

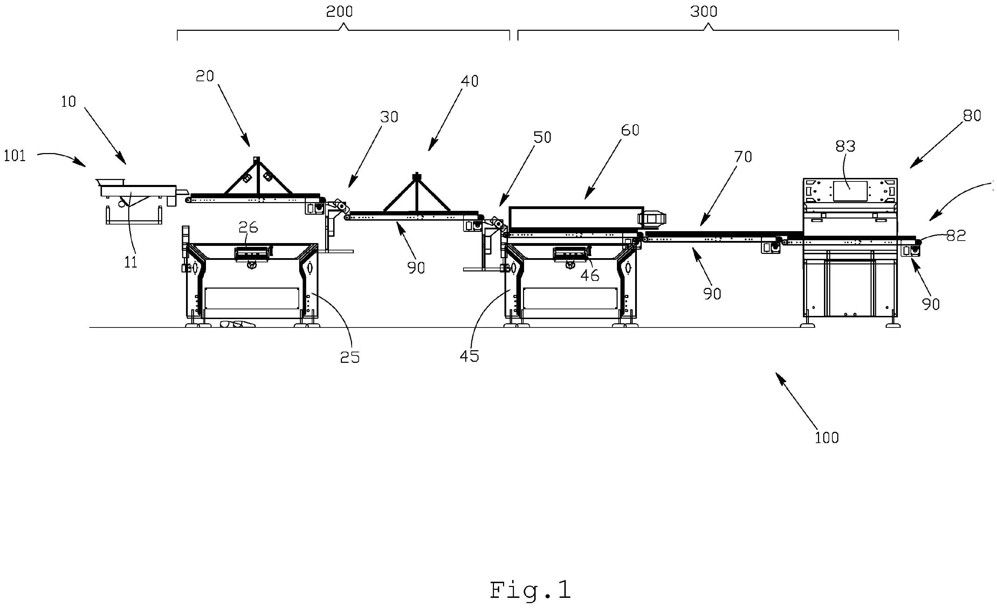

With reference to Fig. 1, there is illustrated as a whole a system 100 for the inspection and treatment, in particular for the sanitisation, of food products contaminated by aflatoxins.

The system 100 comprises a feed unit 10, an inspection apparatus 200 and a treatment block 300.

The inspection apparatus 200 comprises a first inspection unit 20, a first selection unit 30, a second inspection unit 40 and a second selection unit 50.

The treatment block 300 comprises a first treatment unit 60, a second treatment unit 70 and a third treatment unit 80.

Each unit of the system 100 is configured to convey the food products between an inlet 101 and an outlet 102 of the system 100.

Therefore, each unit includes a portion of the conveyor line of the food products, indicated in the figure with the number 90. The conveyor line comprises a supporting surface 91 on which the products are distributed. The conveyor line 90 is essentially formed by belts and optionally by conveyors, chutes or other similar elements.

The feed unit 10 comprises a vibrating screen 11 suitable to distribute the products uniformly on the conveying surface. Said feed unit preferably also comprises a "levelling" system suitable to spread out any accumulations of products into a single uniform layer.

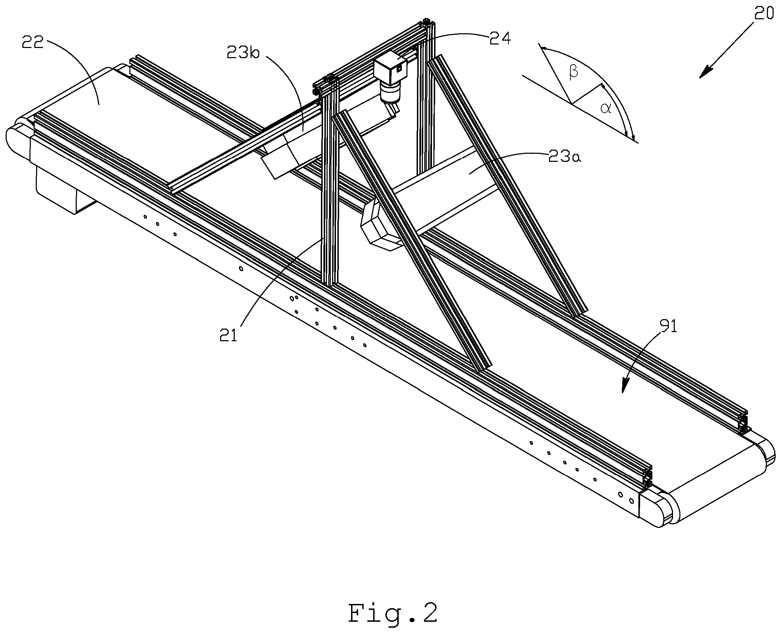

With particular reference to Fig. 2, the first inspection unit 20 of the inspection block 200 comprises a supporting structure 21 positioned above a belt 22 belonging to the conveyor line 90.

Said supporting structure 21 acts as housing for a pair of UV-C lamps 23a, 23b arranged facing each other and for an RGB video camera 24.

The lamps 23a, 23b are arranged so that the light beam is inclined relative to the supporting surface 91 on the belt 22, in particular by an angle α of around 45° for the lamp 23a and by an angle β of around 135° for the lamp 23b, considering the same absolute reference.

The RGB video camera 24 is preferably positioned at the centre of and equidistant from the two lamps 23a, 23b and is oriented substantially perpendicularly to the supporting surface 91.

The+ supporting structure 21 is delimited by walls made of opaque material, not represented in the figures, which prevent exposure of the products to ambient light, which would alter the results of the UV light inspection.

The first inspection unit 20 is associated with a first control station 25 which integrates a control unit dedicated to controlling the lamps 23a, 23b, the video camera 24 and optionally the belt 22. The control unit 25 is preferably equipped with an input/output interface 26, typically a touch screen display, which allows the images filmed by the video camera 24 and/or other information to be viewed and commands to be given to the unit.

A first selection unit 30 connected to the control unit of said first inspection unit 20 is arranged immediately downstream of the first inspection unit 20.

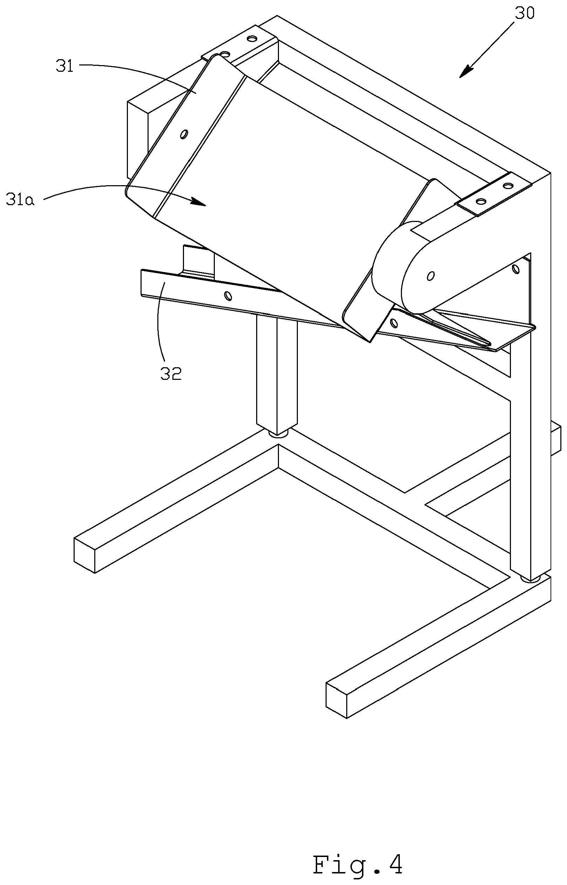

With reference to Fig. 4, the first selection unit 30 comprises a movable channel 31 arranged at the outlet of the belt 22 of the first inspection unit 20 and aligned with said belt 22 in the direction of flow of the food products. Said movable channel 31 has a bottom surface 31a that can receive the flow of food products in movement. The movable channel 31 is, in fact, movable between a first position in which the bottom surface 31a is facing the direction of movement of the flow of products, and a second position in which it is turned in the opposite direction.

In the first position, the movable channel 31 allows the food products to pass from the belt 22 of the first inspection unit 20 to a subsequent station, specifically to the second inspection unit 40, along the conveyor line, as illustrated in Figs. 1 and 4. Instead, in the second position the movable channel 31 acts as diverter to divert the flow of products downward where it is collected from an outlet channel 32.

The movable channel 31 is connected to actuator means, not illustrated in the figure, controlled by the control unit of the first inspection unit 20.

When no contaminated products are detected on the conveyor line, the movable channel remains in the second position and the flow of good products is diverted into the outlet channel 32 toward a collection point.

When the first inspection unit 20 detects potentially contaminated products, the movable channel 31 is moved for a certain interval of time into the first position so as to carry said potentially contaminated products and a certain amount of products in the vicinity toward the second inspection unit 40.

The control station 25 of the first selection unit 20, or rather the control unit comprised therein, is preferably configured also to control said first selection unit 30.

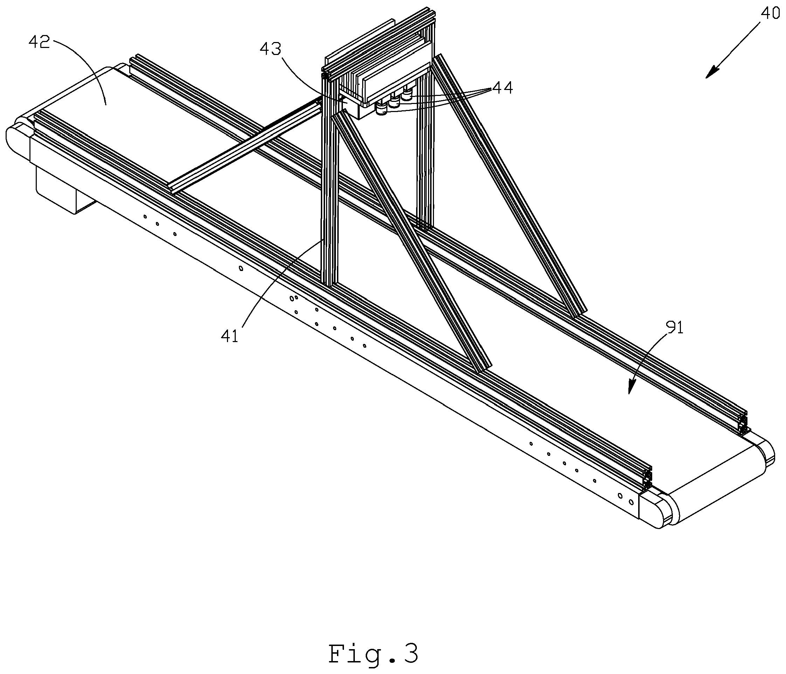

With reference to Fig. 3, the second inspection unit 40, just as the first, comprises a supporting structure 41 positioned above a belt 42 belonging to the conveyor line 90.

The supporting structure 41 acts as housing for a UV-C lamp 43, and for at least one hyperspectral video camera, preferably at least two or three hyperspectral video cameras schematised in the figure and indicated with the reference 44.

The lamp 43 is preferably arranged so that the light beam is substantially perpendicular to the supporting surface 91 on the belt 42.

The hyperspectral video cameras 44 are preferably placed side by side at the centre of the supporting structure 41, aligned or positioned further forward or backward relative to the lamp 43, and are oriented substantially perpendicular to the supporting surface 91.

The second inspection unit 40 is also associated with a second control station 45, which integrates a control unit dedicated to controlling the lamp 43 of the hyperspectral video cameras 44 and optionally of the belt 42 and is equipped with an input/output interface 46.

A second selection unit 50 is arranged downstream of the second inspection unit. Said second selection unit 50 is preferably identical to the first selection unit 30 and has the same function.

Said second selection unit 50 is preferably controllable from the second control station 45.

The products that after inspection in the second inspection unit 40 are found to be still contaminated (or potentially contaminated), are conveyed by the second selection unit 50 toward the treatment block 300.

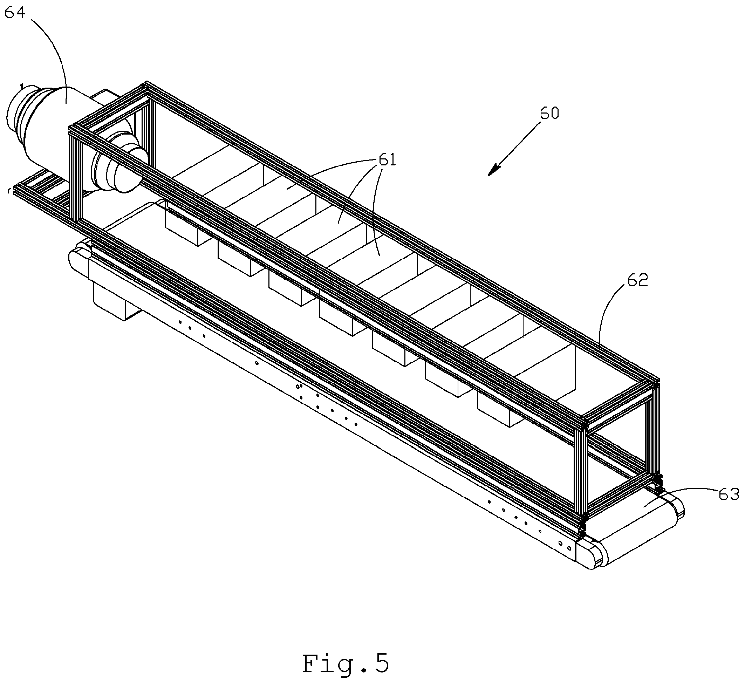

More in detail, in the variant of the system illustrated in Fig. 1, a first treatment unit 60 equipped with ozonator devices 61 is positioned downstream of the second selection unit 50. Said ozonator devices are mounted on a supporting structure 62 that forms a tunnel that is positioned above the belt 63 substantially for the whole of its length, as shown in Fig. 5.

The inner volume in the tunnel 62 is in communication with a suction device 64 suitable to draw in the excess ozone produced by the ozonator devices 61.

The products delivered from the belt 63 of the first treatment unit 60 are conveyed toward the second treatment unit 70, which is equipped with a plurality of UV-C tube lamps 71 arranged parallel along the direction of extension of the respective belt 72. The lamps 71 are mounted on a supporting frame 73 and are placed at a distance from the belt of around 20-40 mm.

According to a preferred variant, the second control station 45 of the second inspection unit 40 is configured also to control the first and the second treatment units. Alternatively, a further control station, common to both said units, or two further stations dedicated to the respective units can be provided.

In the variant illustrated, the treatment system comprises a third ionising radiation treatment unit 80. Said further treatment unit 80 comprises a chamber 81, through which the belt 82 passes, in which an X-ray generator tube (not illustrated in the figure) is placed.

Typically, said third treatment unit 80 is equipped with a dedicated control station 83, preferably integrated in the structure of the unit.

The present invention has been described purely for non-limiting illustrative purposes, according to some preferred embodiments. The person skilled in the art can find numerous other embodiments and variants, all falling within the scope of protection of the appended claims.

## Claims

1. An apparatus (200) for the inspection and selection of food products contaminated by aflatoxins comprising:
- a conveyor line (90) for conveying the food products between an inlet of the apparatus and at least one outlet of the apparatus, said conveyor line (90) having a supporting surface (91) suitable to receive the bulk food products distributed in a uniform layer;
- a first inspection station (20) suitable to inspect a flow of food products in movement, said first inspection station (20) comprising:
- a housing (21) positioned above the supporting surface (91) of the conveyor line (90);
- at least one ultraviolet light source (23a, 23b), mounted in the housing (21) so as to direct a beam of ultraviolet light toward the product on the supporting surface (91);
- an RGB video camera (24) mounted in the housing (21) and oriented toward the area of the supporting surface (91) illuminated by the ultraviolet light source (23a, 23b);
- a first control unit connected at least to the RGB video camera (24), said control unit being configured to receive the images filmed by said video camera (24) and to analyse said images, in particular to identify in the layer of food products in movement on the conveyor line those with photoemissions compatible with that of aflatoxins, and hence potentially contaminated;
- a first selection unit (30), placed downstream of the first inspection unit (20), where said selection unit (30) is controlled by the first control unit and is configured to separate the uncontaminated food products from those potentially contaminated and to eject the former toward a collection point;
the apparatus (200) being **characterised by** the fact that it comprises:
- a second inspection unit (40), placed downstream of the first selection unit (20) along the conveyor line (90), suitable to inspect the flow of potentially contaminated food products, where said second inspection unit (40) comprises:
- a second housing (41) positioned above the supporting surface (91) of the conveyor line (90);
- at least one ultraviolet light source (43), mounted in the second housing (31) so as to direct a beam of ultraviolet light toward the product on the supporting surface (91);
- at least one hyperspectral video camera (44) mounted in the second housing (41) and oriented toward the area of the supporting surface (91) illuminated by the ultraviolet source (43);
- a second control unit, connected at least to the hyperspectral video camera (44), configured to receive the images filmed by said video camera and to analyse said images, in particular to distinguish, based on the wavelengths of the photoemissions of said products, those probably contaminated by aflatoxins from those uncontaminated;
- a second selection unit (50), placed downstream of the second inspection unit (40), said second selection unit (50) being controlled by the second control unit and being configured to separate the uncontaminated food products from those probably contaminated and to eject the former toward a collection point.

2. The apparatus (200) according to claim 1, wherein a pair of ultraviolet light sources (23a, 23b) is arranged in the housing of the first inspection unit (20), facing each other, relative to the direction of movement of the conveyor line (90), so that the light beam of each is oriented with an angle between 35° and 55° or between 125° and 145° relative to the plane of the supporting surface (91).

3. The apparatus (200) according to claim 1, wherein the second inspection unit (40) comprises two or more hyperspectral video cameras (44), each video camera being oriented to frame the same portion of the supporting surface (91) and being configured to detect a specific wavelength or a predefined range of wavelengths.

4. The apparatus (200) according to claim 1 or 3, wherein the second housing (41) of the second inspection unit (40) is equipped with at least one ultraviolet light source (43) arranged substantially perpendicular relative to the plane of the supporting surface (91).

5. The apparatus (200) according to any one of the preceding claims, wherein the at least one ultraviolet light source (23, 23b, 43) is configured to emit light with a wavelength of 365 nm.

6. A system (100) for the treatment of food products, in particular dried fruit, contaminated by aflatoxins, comprising:
- an inspection and selection apparatus (200) according to any one of the preceding claims, suitable to deliver a flow of potentially or probably contaminated food products distributed in a uniform layer on the conveyor line (91);
- at least one treatment unit (60, 70) to eliminate or reduce the aflatoxins present in food products, said treatment unit (60) being positioned above the conveyor line (90), downstream of the inspection and selection apparatus (200);
where said treatment unit (60, 70) comprises:
- a plurality of ozonator devices (61) positioned consecutively along the conveyor line (90) suitable to generate a flow of ozone directed toward the layer of food products; or
- one or more ultraviolet light sources (71) with a wavelength between 100 nm and 280 nm oriented to direct at least a part of the light beam onto the layer of food products.

7. The system (100) according to claim 6, comprising a treatment unit (60) equipped with the ozonator devices (61) and a further treatment unit (70) equipped with the ultraviolet light sources (71).

8. The system (100) according to claim 6 or 7, comprising a further treatment unit (80) comprising an ionising radiation emitting device oriented toward the flow of food products to be treated.

9. The system (100) according to one of claims 6 to 8, comprising a video camera suitable to film the flow of food products entering the treatment unit (60, 70, 80) and a control unit connected to said video camera and configured to control the treatment units (60, 70, 80) in real time as a function of the amount of food product present on the conveyor line (90).

## Patentansprüche

1. Eine Vorrichtung (200) zur Inspektion und Auswahl von Lebensmitteln, die durch Aflatoxine kontaminiert sind, umfassend:
- eine Förderstrecke (90) zum Fördern der Lebensmittel zwischen einem Einlass der Vorrichtung und mindestens einem Auslass der Vorrichtung, wobei die Förderstrecke (90) eine Tragfläche (91) aufweist, die geeignet ist, die in einer gleichmäßigen Schicht verteilten Lebensmittel im Schüttgut aufzunehmen;
- eine erste Inspektionsstation (20), die geeignet ist, einen Strom von bewegten Lebensmitteln zu inspizieren, wobei die erste Inspektionsstation (20) umfasst:
- ein Gehäuse (21), das oberhalb der Tragfläche (91) der Förderstrecke (90) positioniert ist;
- mindestens eine Ultraviolettlichtquelle (23a, 23b), die in dem Gehäuse (21) montiert ist, um einen Strahl von Ultraviolettlicht auf das Produkt auf der Tragfläche (91) zu richten;
- eine RGB-Video-Kamera (24), die in dem Gehäuse (21) montiert ist und auf den Bereich der Tragfläche (91) gerichtet ist, der von der Ultraviolettlichtquelle (23a, 23b) beleuchtet wird;
- eine erste Steuereinheit, die mindestens mit der RGB-Video-Kamera (24) verbunden ist, wobei die Steuereinheit konfiguriert ist, um die von der Video-Kamera (24) aufgenommenen Bilder zu empfangen und die Bilder zu analysieren, insbesondere um in der Schicht von bewegten Lebensmitteln auf der Förderstrecke solche zu identifizieren, die mit denen von Aflatoxinen kompatible Photoemissionen aufweisen, und somit potenziell kontaminiert sind;
- eine erste Auswahleinheit (30), die stromabwärts der ersten Inspektionseinheit (20) platziert ist, wobei die Auswahleinheit (30) von der ersten Steuereinheit gesteuert wird und konfiguriert ist, um die nicht kontaminierten Lebensmittel von den potenziell kontaminierten zu trennen und die ersteren zu einem Sammelort auszuwerfen;
die Vorrichtung (200) **gekennzeichnet durch** die Tatsache, dass sie umfasst:
- eine zweite Inspektionseinheit (40), die stromabwärts der ersten Auswahleinheit (20) entlang der Förderstrecke (90) platziert ist, die geeignet ist, den Strom von potenziell kontaminierten Lebensmitteln zu inspizieren, wobei die zweite Inspektionseinheit (40) umfasst:
- ein zweites Gehäuse (41), das oberhalb der Tragfläche (91) der Förderstrecke (90) positioniert ist;
- mindestens eine Ultraviolettlichtquelle (43), die in dem zweiten Gehäuse (31) montiert ist, um einen Strahl von Ultraviolettlicht auf das Produkt auf der Tragfläche (91) zu richten;
- mindestens eine hyperspektrale Video-Kamera (44), die in dem zweiten Gehäuse (41) montiert ist und auf den Bereich der Tragfläche (91) gerichtet ist, der von der Ultraviolettlichtquelle (43) beleuchtet wird;
- eine zweite Steuereinheit, die mindestens mit der hyperspektralen Video-Kamera (44) verbunden ist, konfiguriert zum Empfangen der von der Video-Kamera aufgenommenen Bilder und zum Analysieren der Bilder, insbesondere zum Unterscheiden, basierend auf den Wellenlängen der Photoemissionen der Produkte, solcher, die wahrscheinlich durch Aflatoxine kontaminiert sind, von solchen, die nicht kontaminiert sind;
- eine zweite Auswahleinheit (50), die stromabwärts der zweiten Inspektionseinheit (40) platziert ist, wobei die zweite Auswahleinheit (50) von der zweiten Steuereinheit gesteuert wird und konfiguriert ist, um die nicht kontaminierten Lebensmittel von den wahrscheinlich kontaminierten zu trennen und die ersteren zu einem Sammelort auszuwerfen.

2. Die Vorrichtung (200) nach Anspruch 1, wobei ein Paar von Ultraviolettlichtquellen (23a, 23b) in dem Gehäuse der ersten Inspektionseinheit (20) angeordnet ist, einander gegenüberliegend, relativ zur Bewegungsrichtung der Förderstrecke (90), so dass der Lichtstrahl jedes Einzelnen mit einem Winkel zwischen 35° und 55° oder zwischen 125° und 145° relativ zur Ebene der Tragfläche (91) gerichtet ist.

3. Die Vorrichtung (200) nach Anspruch 1, wobei die zweite Inspektionseinheit (40) zwei oder mehr hyperspektrale Video-Kameras (44) umfasst, wobei jede Video-Kamera so gerichtet ist, um denselben Abschnitt der Tragfläche (91) einzufangen und konfiguriert ist, um eine bestimmte Wellenlänge oder einen vordefinierten Bereich von Wellenlängen zu detektieren.

4. Die Vorrichtung (200) nach Anspruch 1 oder 3, wobei das zweite Gehäuse (41) der zweiten Inspektionseinheit (40) mit mindestens einer Ultraviolettlichtquelle (43) ausgestattet ist, die im Wesentlichen senkrecht relativ zur Ebene der Tragfläche (91) angeordnet ist.

5. Die Vorrichtung (200) nach einem der vorhergehenden Ansprüche, wobei die mindestens eine Ultraviolettlichtquelle (23, 23b, 43) konfiguriert ist, um Licht mit einer Wellenlänge von 365 nm zu emittieren.

6. Ein System (100) zur Behandlung von Lebensmitteln, insbesondere von Trockenfrüchten, die durch Aflatoxine kontaminiert sind, umfassend:
- eine Inspektions- und Auswahlvorrichtung (200) nach einem der vorhergehenden Ansprüche, die geeignet ist, einen Strom von potenziell oder wahrscheinlich kontaminierten Lebensmitteln in einer gleichmäßigen Schicht auf der Förderstrecke (91) zu liefern;
- mindestens eine Behandlungseinheit (60, 70) zum Eliminieren oder Reduzieren der in Lebensmitteln vorhandenen Aflatoxine, wobei die Behandlungseinheit (60) oberhalb der Förderstrecke (90), stromabwärts der Inspektions- und Auswahlvorrichtung (200), positioniert ist;
wobei die Behandlungseinheit (60, 70) umfasst:
- eine Vielzahl von Ozonatorvorrichtungen (61), die nacheinander entlang der Förderstrecke (90) positioniert sind und geeignet sind, einen Ozonstrom zu erzeugen, der auf die Schicht von Lebensmitteln gerichtet ist; oder
- eine oder mehrere Ultraviolettlichtquellen (71) mit einer Wellenlänge zwischen 100 nm und 280 nm, die so gerichtet sind, um mindestens einen Teil des Lichtstrahls auf die Schicht von Lebensmitteln zu richten.

7. Das System (100) nach Anspruch 6, umfassend eine Behandlungseinheit (60), die mit den Ozonatorvorrichtungen (61) ausgestattet ist, und eine weitere Behandlungseinheit (70), die mit den Ultraviolettlichtquellen (71) ausgestattet ist.

8. Das System (100) nach Anspruch 6 oder 7, umfassend eine weitere Behandlungseinheit (80), die eine ionisierende Strahlung emittierende Vorrichtung umfasst, die auf den Strom von zu behandelnden Lebensmitteln gerichtet ist.

9. Das System (100) nach einem der Ansprüche 6 bis 8, umfassend eine Video-Kamera, die geeignet ist, den Strom von Lebensmitteln zu filmen, der in die Behandlungseinheit (60, 70, 80) eintritt, und eine Steuereinheit, die mit der Video-Kamera verbunden ist und konfiguriert ist, um die Behandlungseinheiten (60, 70, 80) in Echtzeit in Abhängigkeit von der auf der Förderstrecke (90) vorhandenen Menge an Lebensmitteln zu steuern.

## Revendications

1. Un appareil (200) pour l'inspection et la sélection de produits alimentaires contaminés par des aflatoxines comprenant:
- une ligne de convoyage (90) pour convoyer les produits alimentaires entre une entrée de l'appareil et au moins une sortie de l'appareil, ladite ligne de convoyage (90) ayant une surface de support (91) appropriée pour recevoir les produits alimentaires en vrac distribués en une couche uniforme;
- une première station d'inspection (20) appropriée pour inspecter un flux de produits alimentaires en mouvement, ladite première station d'inspection (20) comprenant:
- un logement (21) positionné au-dessus de la surface de support (91) de la ligne de convoyage (90);
- au moins une source de lumière ultraviolette (23a, 23b), montée dans le logement (21) de manière à diriger un faisceau de lumière ultraviolette vers le produit sur la surface de support (91);
- une caméra vidéo RGB (24) montée dans le logement (21) et orientée vers la zone de la surface de support (91) éclairée par la source de lumière ultraviolette (23a, 23b);
- une première unité de commande connectée au moins à la caméra vidéo RGB (24), ladite unité de commande étant configurée pour recevoir les images filmées par ladite caméra vidéo (24) et pour analyser lesdites images, en particulier pour identifier dans la couche de produits alimentaires en mouvement sur la ligne de convoyage ceux ayant des photoémissions compatibles avec celles des aflatoxines, et donc potentiellement contaminés;
- une première unité de sélection (30), placée en aval de la première unité d'inspection (20), où ladite unité de sélection (30) est contrôlée par la première unité de commande et est configurée pour séparer les produits alimentaires non contaminés de ceux potentiellement contaminés et pour éjecter les premiers vers un point de collecte;
l'appareil (200) étant **caractérisé par le fait qu'**il comprend:
- une deuxième unité d'inspection (40), placée en aval de la première unité de sélection (20) le long de la ligne de convoyage (90), appropriée pour inspecter le flux de produits alimentaires potentiellement contaminés, où ladite deuxième unité d'inspection (40) comprend:
- un deuxième logement (41) positionné au-dessus de la surface de support (91) de la ligne de convoyage (90);
- au moins une source de lumière ultraviolette (43), montée dans le deuxième logement (31) de manière à diriger un faisceau de lumière ultraviolette vers le produit sur la surface de support (91);
- au moins une caméra vidéo hyperspectrale (44) montée dans le deuxième logement (41) et orientée vers la zone de la surface de support (91) éclairée par la source ultraviolette (43);
- une deuxième unité de commande, connectée au moins à la caméra vidéo hyperspectrale (44), configurée pour recevoir les images filmées par ladite caméra vidéo et pour analyser lesdites images, en particulier pour distinguer, sur la base des longueurs d'onde des photoémissions desdits produits, ceux probablement contaminés par des aflatoxines de ceux non contaminés;
- une deuxième unité de sélection (50), placée en aval de la deuxième unité d'inspection (40), ladite deuxième unité de sélection (50) étant contrôlée par la deuxième unité de commande et étant configurée pour séparer les produits alimentaires non contaminés de ceux probablement contaminés et pour éjecter les premiers vers un point de collecte.

2. L'appareil (200) selon la revendication 1, dans lequel une paire de sources de lumière ultraviolette (23a, 23b) est disposée dans le logement de la première unité d'inspection (20), faisant face l'une à l'autre, par rapport à la direction de mouvement de la ligne de convoyage (90), de sorte que le faisceau lumineux de chacune est orienté avec un angle compris entre 35° et 55° ou entre 125° et 145° par rapport au plan de la surface de support (91).

3. L'appareil (200) selon la revendication 1, dans lequel la deuxième unité d'inspection (40) comprend deux ou plusieurs caméras vidéo hyperspectrales (44), chaque caméra vidéo étant orientée pour cadrer la même portion de la surface de support (91) et étant configurée pour détecter une longueur d'onde spécifique ou une plage prédéfinie de longueurs d'onde.

4. L'appareil (200) selon la revendication 1 ou 3, dans lequel le deuxième logement (41) de la deuxième unité d'inspection (40) est équipé d'au moins une source de lumière ultraviolette (43) disposée substantiellement perpendiculairement par rapport au plan de la surface de support (91).

5. L'appareil (200) selon l'une quelconque des revendications précédentes, dans lequel l'au moins une source de lumière ultraviolette (23, 23b, 43) est configurée pour émettre de la lumière avec une longueur d'onde de 365 nm.

6. Un système (100) pour le traitement de produits alimentaires, en particulier des fruits secs, contaminés par des aflatoxines, comprenant :
- un appareil d'inspection et de sélection (200) selon l'une quelconque des revendications précédentes, approprié pour délivrer un flux de produits alimentaires potentiellement ou probablement contaminés distribués en une couche uniforme sur la ligne de convoyage (91);
- au moins une unité de traitement (60, 70) pour éliminer ou réduire les aflatoxines présentes dans les produits alimentaires, ladite unité de traitement (60) étant positionnée au-dessus de la ligne de convoyage (90), en aval de l'appareil d'inspection et de sélection (200);
où ladite unité de traitement (60, 70) comprend:
- une pluralité de dispositifs ozonateurs (61) positionnés consécutivement le long de la ligne de convoyage (90) appropriés pour générer un flux d'ozone dirigé vers la couche de produits alimentaires; ou
- une ou plusieurs sources de lumière ultraviolette (71) avec une longueur d'onde comprise entre 100 nm et 280 nm orientées pour diriger au moins une partie du faisceau lumineux sur la couche de produits alimentaires.

7. Le système (100) selon la revendication 6, comprenant une unité de traitement (60) équipée des dispositifs ozonateurs (61) et une autre unité de traitement (70) équipée des sources de lumière ultraviolette (71).

8. Le système (100) selon la revendication 6 ou 7, comprenant une autre unité de traitement (80) comprenant un dispositif émetteur de rayonnement ionisant orienté vers le flux de produits alimentaires à traiter.

9. Le système (100) selon l'une des revendications 6 à 8, comprenant une caméra vidéo appropriée pour filmer le flux de produits alimentaires entrant dans l'unité de traitement (60, 70, 80) et une unité de commande connectée à ladite caméra vidéo et configurée pour contrôler les unités de traitement (60, 70, 80) en temps réel en fonction de la quantité de produit alimentaire présente sur la ligne de convoyage (90).
